# FASCICULE DE BREVET EUROPEEN

(11) **EP 0 323 307 B1**
(45) Date de publication et mention de la délivrance du brevet: **14.04.1993**
(21) Numéro de dépôt: 88403205.3
(22) Date de dépôt: 16.12.1988
(51) Int. Cl.: F17D 1/05, C10L 3/00

(54) **Procédé de transport d'un fluide formant des hydrates**
Transportverfahren eines Fluidums unter Hydratbildung
Process for transporting a fluid which forms hydrates

(30) Priorité: 30.12.1987 FR 8718435
(43) Date de publication de la demande: 05.07.1989
(73) Titulaire: INSTITUT FRANCAIS DU PETROLE, 92502 Rueil-Malmaison (FR)
(72) Inventeur: Sugier, André, F-92500 Rueil Malmaison (FR); Bourgmayer, Paul, F-92500 Rueil Malmaison (FR); Behar, Emmanuel, F-95000 Cergy (FR); Freund, Edouard, F-92500 Rueil Malmaison (FR)

(56) Documents cités:
- EP-A- 0 087 711
- US-A- 2 386 182
- US-A- 3 644 107
- US-A- 4 099 537
- US-A- 4 132 535
- PATENT ABSTRACTS OF JAPAN, vol. 8, no. 120 (M-300)[1557], 6 juin 1984, page 143 M 300.
- Chemical Abstracts vol. 80 (1974), no. 98122 r

## Description

L'invention concerne un procédé pour transporter un fluide comportant du gaz et de l'eau, le fluide étant dans des conditions où au moins un hydrate se forme. Les gaz, tels que le gaz naturel, le gaz de pétrole ou d'autres gaz, qui forment les hydrates avec l'eau, peuvent notamment comprendre, par exemple, du méthane, de l'éthane, de l'éthylène, du propane, du propène, du n-butane, de l'i-butane, de l'H₂S et/ou du CO₂.

Ces hydrates se forment lorsque de l'eau se trouve en présence du gaz, soit à l'état libre, soit à l'état dissous dans une phase liquide, telle qu'un hydrocarbure liquide, et lorsque la température atteinte par le mélange notamment d'eau, de gaz et éventuellement d'hydrocarbures liquides, tels que de l'huile, devient inférieure à la température thermodynamique de formation des hydrates, cette température étant donnée pour une composition des gaz connue et lorsque leur pression est fixée.

La formation d'hydrates peut être redoutée, notamment dans l'industrie pétrolière et gazière, pour lesquelles les conditions de formation d'hydrates peuvent être réunies. En effet, pour diminuer le coût de production du pétrole brut et du gaz, tant au point de vue des investissements qu'au point de vue de l'exploitation, une voie envisagée, notamment en production en mer, est de réduire, voire de supprimer, les traitements appliqués au brut ou au gaz à transporter du gisement à la côte et notamment de laisser toute ou une partie de l'eau dans le fluide à transporter. Ces traitements en mer s'effectuent en général sur une plate-forme située en surface à proximité du gisement, de manière que l'effluent, initialement chaud, puisse être traité avant que les conditions thermodynamiques de formation des hydrates ne soient atteintes du fait du refroidissement de l'effluent avec l'eau de mer.

Cependant, comme cela arrive pratiquement, lorsque les conditions thermodynamiques requises pour former des hydrates sont réuni es, l'agglomération des hydrates entraîne le remplissage et le blocage des conduites de transport par création de bouchons qui empêchent tout passage de pétrole brut ou de gaz.

La formation de bouchons d'hydrates peut entraîner un arrêt de la production et provoquer ainsi des pertes financières importantes. De plus, la remise en service de l'installation, surtout s'il s'agit de production ou de transport en mer, peut être très longue, car la décomposition des hydrates formés est très difficile à réaliser. En effet, lorsque la production d'un gisement sous-marin de gaz naturel ou de pétrole et de gaz comportant de l'eau atteint la surface du sol marin, et est ensuite transportée au fond de la mer, il arrive par l'abaissement de la température de l'effluent produit, que les conditions thermodynamiques soient réunies pour que des hydrates se forment, s'agglomèrent et bloquent les conduites de transfert. La température au fond de la mer peut être par exemple de 3 ou 4°C.

Des conditions favorables à la formation d'hydrates peuvent aussi être réunies de la même façon à terre, pour des conduites pas ou trop faiblement enfouies dans le sol terrestre, lorsque par exemple la température de l'air ambiant est trop froide.

Pour éviter ces inconvénients, on a cheché dans l'art anterieur à utiliser des produits qui, ajoutés au fluide, pourraient agir comme inhibiteurs en abaissant la température thermodynamique de formation des hydrates.

Ainsi, Kuliev et al dans "Surfactants studied as hydrate-formation inhibitors" Gazovoe Delo n^{o} 10 1972, 17-19, rapporté dans Chemical Abstracts 80, 1974 98122 r, ont testé divers composés tensio-actifs non-ioniques ou anioniques pour retarder la formation d'hydrates à l'intérieur d'un fluide renfermant un gaz notamment un hydrocarbure, et de l'eau.

On a également préconisé l'isolation des conduits de transport, de manière à éviter que la température du fluide transporté n'atteigne la température de formation des hydrates dans les conditions opératoires.

Ces deux solutions sont très onéreuses car, pour la première, la quantité d'inhibiteurs à ajouter peut atteindre 10 à 20 % de la teneur en eau et ces inhibiteurs sont difficiles à récupérer complètement ; et pour la seconde des solutions, l'isolation de la conduite est, elle aussi, très coûteuse.

Il a été découvert que certains composés amphiphiles ajoutés au fluide, qui jusqu'à présent n'étaient pas utilisés dans ce but, présentent une excellente efficacité pour modifier le mécanisme de formation de ces hydrates. Cette modification du mécanisme peut notamment et avantageusement être mise à profit pour le transport de fluide générant des hydrates.

L'invention propose donc un procédé de transport dans une conduite d'un fluide comportant du gaz et de l'eau, et étant dans des conditions où au moins un hydrate se forme, les hydrates étant formés à partir dudit gaz et de ladite eau, caractérisé en ce qu'on injecte dans ledit fluide, avant ou pendant la formation du ou des hydrates, un additif comprenant au moins un composé tensio-actif adapté à réduire la tendance à l'agglomération des hydrates de manière à obtenir un ou des hydrates sous forme dispersée, et on ce qu'on transporte ledit fluide contenant ce ou ces hydrates sous forme dispersée.

Les composés amphiphiles sont des composés chimiques comportant une partie hydrophile ou polaire et une partie oléophile ou lipophile.

En effet, au lieu de constater que les hydrates s'agglomèrent les uns aux autres en formant des blocs et des bouchons très solides, ou des dépôts dans les appareils où circule le fluide générant des hydrates, il a été observé, dans une large plage de température, que ces composés dispersent ces hydrates dans ce fluide et empêchent alors leur agglomération.

Lorsqu'on soumet un mélange de gaz, de fluides et de certains composés amphiphiles à une température notablement plus basse que la température de début de formation des hydrates, il se crée un épaississement du fluide, sans qu'il y ait de formation de blocs ou de bouchons, cet épaississement étant d'autant plus fort que la température est basse. Cet épaississement du fluide est dû d'une part à l'accroissement de la viscosité de fluide sous l'effet de la température, et d'autre part, à la présence de particules d'hydrates sous forme dispersée.

Des essais en dynamique utilisant certains composés amphiphiles ont alors permis de constater que le transport de fluide saturé, ou en voie de saturation, d'hydrates est possible.

Outre cette aptitude à disperser les hydrates qui se forment dans le fluide, les composés amphiphiles observés peuvent abaisser la température de début de formation des hydrates, plus ou moins selon leur concentration, et diminuent le coût de transport de fluides susceptibles de former des hydrates du fait des faibles quantités des produits utilisés (généralement inférieures à 1 % poids par rapport à l'eau) et du coût unitaire modéré de ceux-ci.

Ces composés amphiphiles, ou plus généralement ces additifs utilisés seuls, en mélange, ou en présence d'autres composés (méthanol, glycol), contenant ces composés que, selon l'invention, on utilise pour transporter un fluide générant ou allant générer des hydrates de gaz, sont choisis, par exemple, parmi les composés amphiphiles non-ioniques, les composés amphiphiles anioniques, et les composés amphiphiles cationiques.

Les composés amphiphiles non-ioniques sont caractérisés en ce qu'ils comportent :
- une partie hydrophile comportant des groupes soit oxyde d'alkylène, soit hydroxyles, soit encore alkylène amine,
- une partie oléophile comportant une chaîne hydrocarbonnée dérivée d'un alcool, d'un acide gras, d'un dérivé alkylé du phénol, ou d'une polyoléfine par exemple à base d'isobutène ou de butènes, et
- une liaison entre la partie hydrophile et la partie oléophile qui peut être par exemple un pont éther, ester ou amide ; la liaison peut encore être obtenue par un atome d'azote ou de soufre.

Parmi les composés amphiphiles non-ioniques, on peut citer les alcools gras oxyéthylés, les alkylphénols alcoxylés, les dérivés oxyéthylés et/ou oxypropylés, les éthers de sucre, les esters de polyols, tels que le glycérol, le polyéthylèneglycol, le sorbitol ou le sorbitan, les esters de sucre, les mono et diéthanolamides, les amides d'acides carboxyliques, d'acides sulfoniques ou d'acides aminés.

Les composés amphiphiles anioniques sont caractérisés en ce qu'ils comportent un ou plusieurs groupements fonctionnels s'ionisant en solution aqueuse pour fournir des ions chargés négativement et responsables de l'activité de surface. Ce groupement fonctionnel est un groupement acide salifié par un métal ou une amine. L'acide peut être par exemple carboxylique, sulfonique, sulfurique, etc.

Parmi les composés amphiphiles anioniques, on peut citer :
- les carboxylates, tels les savons métalliques, les savons alcalins ou les savons organiques (comme les N-acylaminoacides, les N-acylsarcosinates, les N-acylglutamates, les N-acylpolypeptides),
- les sulfonates, tels que les alkybenzènesulfonates, comme les alkylbenzènesulfonates alcoxylés, les paraffinesulfonates, les oléfinesulfonates, les petroleum sulfonates, les lignosulfonates ou les dérivés sulfosucciniques (comme les sulfosuccinamates, les hémisulfosuccinates, les dialkylsulfosuccinates, tels que le dioctylsulfosuccinate de sodium),
- les sulfates, tels que les alkylsulfates, les alkyléthersulfates,
- les phosphates.

Les composés amphiphiles cationiques sont caractérisés en ce qu'ils comportent un ou plusieurs groupements fonctionnels s'ionisant en solution aqueuse pour founir des ions chargés positivement et responsables de l'activité de surface.

Parmi les composés amphiphiles cationiques, on peut citer, les sels d'alkylamines, tels que les alkylaminéthers, les sels d'ammonium quaternaires, tels que les dérivés d'alkyltriméthylammonium, les dérivés d'alkyltriéthylammonium, les dérivés d'alkyldiméthylbenzylammonium, les dérivés d'alkylamine alcoxylés, les dérivés hétérocycliques, tels que les dérivés de pyridinium, d'imidazolinium, de quinolinium, de piperidinium ou de morpholinium.

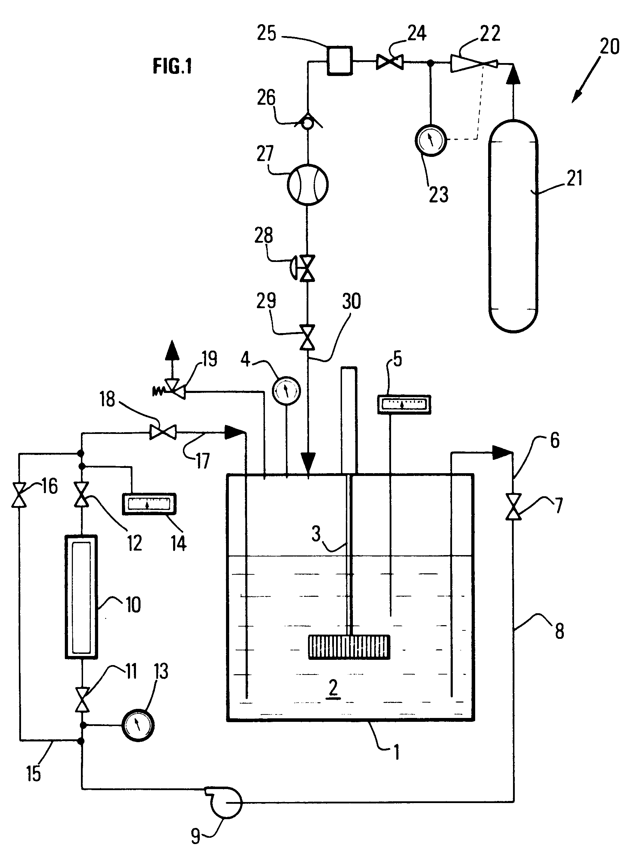

D'une part pour simuler le transport de fluide formant des hydrates, tels que des effluents pétroliers, et s'apercevoir de l'action dispersante de certains additifs sur les hydrates, et d'autre part pour évaluer l'efficacité des additifs, on a procédé à des essais de formation d'hydrates à partir de gaz, de condensat et d'eau à l'aide d'un appareillage schématisé par la figure 1.

L'appareillage comporte un réacteur 1 thermorégulé, de 2 litres de volume, dans lequel est placé un liquide 2, tel un mélange de condensat et d'eau, qui est agité en permanence par un agitateur 3 monté en bout d'une turbine. L'alimentation en gaz du réacteur 1 est régulé par un manomètre 4, la température du réacteur et de la boucle de circulation est contrôlée au moyen de bains thermostatiques dont la température est régulée par la sonde de température 5. Une canalisation 6 plongeant d'un côté dans le liquide 2 alimente par son autre côté une boucle de circulation 8 que l'on peut fermer par la vanne 7.

Sur la boucle de circulation 8 est placée une pompe 9 assurant la circulation du fluide et du gaz. La boucle 8 comporte en outre une chambre d'observation 10, isolable par deux vannes 11 et 12, dans laquelle on peut observer la formation d'hydrates.

En amont et aval de cette chambre se trouvent respectivement un indicateur de pression 13 et un indicateur de température 14. L'appareillage comporte une dérivation 15 de la chambre d'observation, cette dérivation étant munie d'une vanne d'arrêt 16.

Le fluide et le gaz, ayant traversé la chambre d'observation 10 ou la dérivation 15, rejoignent le réacteur 1 par une canalisation de retour 17. Une vanne 18 permet d'isoler le circuit de la canalisation de retour. Le réacteur 1 comporte en outre une soupape de sécurité 19.

L'alimentation en gaz du réacteur 1 est réalisée par le circuit désigné 20 dans son ensemble et qui comprend, montés à la suite des uns des autres, les éléments suivants : un réservoir 21 de gaz, un détendeur 22, un manomètre 23 à pression consignée commandant le détendeur 22, une vanne de fermeture 24, un filtre 25, un clapet antiretour 26, un débitmètre 27, une vanne électronique 28 commandée par le manomètre 4 et assurant la consigne de pression à l'intérieur du réacteur par modification du flux gazeux, une vanne d'arrêt 29 et une canalisation d'alimentation 30 pénétrant dans le réacteur.

Dans un exemple de réalisation, la boucle de circulation 8 fait 10 mètres de longueur et est réalisée en tube d'environ 19 mm (3/4") de diamètre intérieur. La pompe de circulation 9 permet de réaliser des vitesses d'écoulement jusqu'à 1m/s.

La formation d'hydrates par réaction du gaz avec l'eau, se traduit par une consommation de gaz qui est déterminée par le débitmètre 27, et qui est contrôlée par la vanne électrique 28 et le capteur différentiel de pression 23, de manière que la pression soit maintenue constante dans le circuit au 1/50ème de bar près.
L'expérimentation est conduite sous une pression de 7 MPa maintenue constante par apport de gaz.

Pour déterminer la température à laquelle se forment les hydrates, on réalise à partir de la température ambiante, une descente rapide en température de 3°C par heure jusqu'à 1°C.

Ayant alors noté la température de début de formation des hydrates, qui se traduit par une consommation du gaz, on remonte la température du réacteur et de la bouche de circulation de 5°C au-dessus de cette température de formation et on attend que la décomposition des hydrates soit complète. Cette décomposition se manifeste par un accroissement de la pression dans le réacteur 1 et par le disparition visuelle de l'opacité du fluide qui est produite par la présence d'hydrates.

Enfin, on réalise une descente lente en température à raison de 1°C/heure et l'on détermine la température à laquelle les hydrates commencent à se former, puis on détermine la température à laquelle le circuit est totalement bouché et où aucune circulation du fluide n'est possible.

Les exemples suivants illustrent, de manière non limitative, l'utilisation de quelques additifs dans le procédé selon l'invention de transport de fluide formant des hydrates. Les exemples 1 et 2 sont donnés à titre comparatif.

### Exemple n^{o} 1

Dans cet exemple, on opère avec un fluide composé en volume de 20 % d'eau et de 80 % de condensat. La composition pondérale du condensat est : pour les molécules ayant moins de 11 atomes de carbone : 20 % de paraffines et d'isoparaffines, 48 % de naphtènes, 10 % d'aromatiques : et pour les molécules ayant au moins 11 atomes de carbone : 22 % d'un mélange de paraffines, d'isoparaffines, de naphtènes et d'aromatiques.

Le gaz utilisé comprend en volume 98 % de méthane et 2 % d'éthane. L'expérimentation est conduite sous une pression de 7MPa maintenue constante par rapport de gaz. Dans ces conditions, la température de début de formation d'hydrates, lors de la deuxième descente en température, est de 11,4°C, et on obtient un blocage de la circulation par croissance et coalescence des hydrates lorsque la température atteint + 11°C, soit 24 minutes après le début de formation des hydrates.

### Exemple n^{o} 2

Dans cet exemple, on opère comme dans l'exemple n^{o}1, avec le même fluide, le même gaz et la même pression, mais on ajoute au fluide en circulation 5 % poids de méthanol par rapport à l'eau du mélange, le méthanol étant le plus couramment employé pour le transport des effluents pétroliers lorsqu'il y a des risques de formation d'hydrates. Dans ces conditions, on constate que la température à laquelle les hydrates commençent à se former est de 9,4°C et que la température à laquelle aucune circulation de fluide n'est plus possible, du fait de la croissance et la coalescence des hydrates, est de 9°C.

### Exemple n^{o} 3

On opère comme dans l'exemple n^{o}1, mais on ajoute au fluide en circulation 0,25 % poids par rapport à l'eau de diéthanolamides du coprah.

Dans ces conditions on constate que la température à laquelle les hydrates commencent à se former est de 7,5°C et qu'à -10°C, température minimale à laquelle on a opéré, aucun blocage du fluide en circulation n'a été constaté.

### Exemple n^{o} 4

On opère comme dans l'exemple n^{o}3 avec 0,25 % poids de diéthanolamides de coprah, mais lorsque l'on atteint - 2°C, température à laquelle il n'y a pas blocage, on arrête la circulation du fluide et au bout d'une heure d'arrêt on remet la pompe en service pendant 2 minutes pour voir s'il y a blocage.

Dans ces conditions, on constate, au bout de 24 heures à - 2°C qu'aucun blocage ne s'est produit et que chaque fois que la pompe est remise en service, toutes les heures, la circulation du fluide contenant les hydrates s'effectue normalement.

### Exemple n^{o} 5

On opère comme dans l'exemple n^{o}1, mais on ajoute au fluide en circulation 0,2 % poids par rapport à l'eau de diéthanolamides d'huile de colza.

Dans ces conditions, la température de début de formation des hydrates est de 8,3°C, et l'on constate qu'à - 5°C aucun blocage du fluide en circulation ne s'est produit.

### Exemple n^{o} 6

On opère comme dans l'exemple n^{o}1, mais on ajoute au fluide en circulation 0,1 % en poids, par rapport à l'eau, de diéthanolamides de beurre.
Dans ces conditions, on constate que la température de formation des hydrates est de 10°C et que la température à laquelle il y a un blocage de la circulation de fluide est de + 3°C.

### Exemple n^{o} 7

On opère comme dans l'exemple n^{o} 1, mais on ajoute au fluide en circulation 0,5 % poids de dioctyl sulforuccinate de sodium à 65 % de concentration en poids.

Dans ces conditions, on constate que la température de début de formation des hydrates est de 9,5°C et que la température à laquelle il y a blocage de la circulation de fluide est de + 7,5°C.

### Exemple n^{o} 8

On opère comme dans l'exemple n^{o} 1, mais on ajoute au fluide en circulation 0,2 % poids par rapport à l'eau de monolaurate de sorbitan.

Dans ces conditions, on constate que la température de début de formation des hydrates est de 9,7°C et qu'il y a blocage de la circulation de fluide à une température de + 5°C.

### Exemple n^{o} 9

On opère comme dans l'exemple n^{o} 1, mais on ajoute au fluide en circulation 0,2 % poids par rapport à l'eau d'un mélange de 80 % poids de monolaurate de sorbitan et de 20 % poids de disoctylsulfosuccinate de sodium à 65 % de concentration en poids.

Dans ces conditions, on constate que la température à laquelle les hydrates commencent à se former est de 9,3°C et qu'il y a blocage de la circulation du fluide à une température de 4,5°C.

Dans les exemples n^{o}1 et 2, en présence de méthanol seulement, ou avec le fluide à tester seul, on observe un blocage de la boucle et cela très rapidement après le début de formation des hydrates, soit 0,4°C au-dessous de la température de début de formation, soit 24 minutes après avoir atteint cette température, temps nécessaire à la coalescence et à la croissance des hydrates.
Par contre, dans les exemples 3, 4 et 5, pour les températures d'essai en-dessous de la température de début de formation d'hydrates, pour les exemples 6 à 9 entre la température de début de formation d'hydrates et la température de blocage de la circulation de fluide, on observe que les hydrates forment des cristaux dispersés dans le fluide et que l'on peut transporter ces cristaux sans bloquer la circulation de fluide.

Les exemples 7, 8 et 9 montrent la synergie produite par l'association d'un composé amphiphile anionique (dioctylsulfosuccinate de sodium) et d'un composé amphiphile non-ionique (monolaurate de sorbitan) pour retarder la formation d'hydrates et réaliser la dispersion de ceux-ci à l'intérieur du fluide, lorsqu'ils se sont formés.

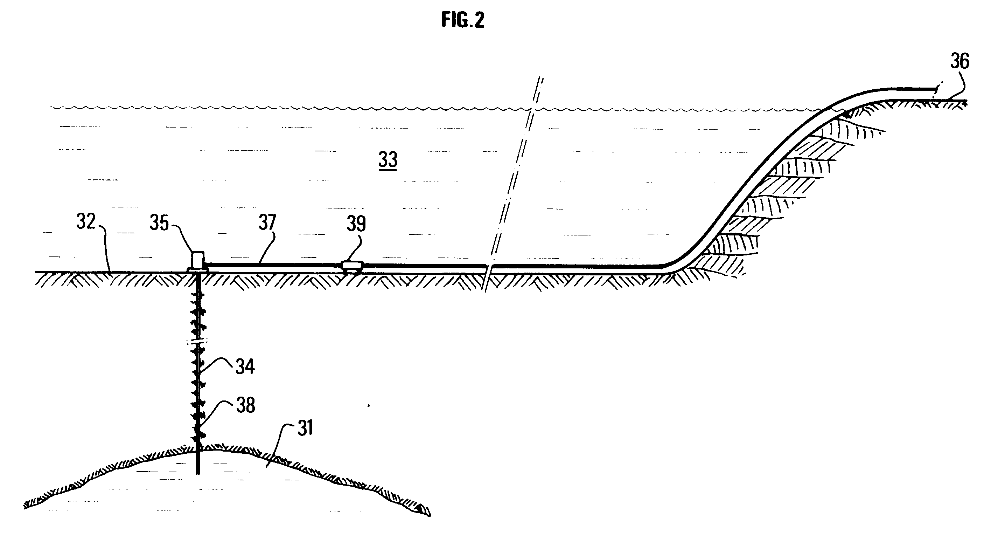

Appliqué notamment aux effluents pétroliers, le procédé de transport selon l'invention sera bien compris et ses avantages apparaîtront clairement à la lecture d'un exemple de réalisation illustré par la figure 2 représentant la production et le transport sous-marin de ces effluents formant des hydrates.

Cet effluent pétrolier formant des hydrates, qui peut être par exemple soit un pétrole brut contenant du gaz et de l'eau, soit du gaz naturel et de l'eau, provient de formations géologiques 31 disposées sous le fond 32 de la mer 33 et exploitées par un puits 34. L'extrêmité supérieure de ce puits 34 ou tête de puits 35, qui est sensiblement disposée au niveau du fond 32 de la mer, est reliée par une conduite 37 placée sur le fond 32 de la mer à des installations de stockage ou de traitement disposées sur les terres émergées 36. Cette conduite 37 peut faire quelques dizaines ou quelques centaines de kilomètres de longueur.

Lorsque la pression de l'effluent présent dans les formations géologiques à produire n'est pas suffisante, soit pour extraire l'effluent de formations, soit pour transporter l'effluent jusqu'aux terres émergées, on place, dans le puits 34 à la hauteur hydraulique nécessaire et/ou sur la conduite 37, respectivement une pompe de puits 38 et/ou une pompe 39 de fond de mer.

D'autre part, comme la température du fond de la mer peut atteindre 3°C, que l'effluent est en contact thermique avec la mer au travers de la paroi de la conduite, la température de l'effluent, qui est initialement élevée lorsqu'il est dans le puits, finit par rejoindre la température de la mer, et quand d'autre part, la composition de l'effluent et la pression sont propices à la formation d'hydrates, ces hydrates s'agglomèrent, forment des bouchons et obstruent la conduite.

Pour pallier cela, le procédé selon l'invention propose
· d'injecter dans l'effluent (ou le fluide formant des hydrates) avant ou pendant la formation de ces hydrates, un additif adapté à réduire la tendance à l'agglomération des hydrates, de manière à obtenir un ou des hydrates sous forme dispersée, et

- de transporter l'effluent contenant ce ou ces hydrates sous forme dispersée.

D'une façon avantageuse, comme il est illustré à la figure 2, on pourra injecter l'additif en amont d'une des pompes 38 ou 39, le plus en amont possible du lieu où l'effluent est dans des conditions où se forment les hydrates.

On pourra aussi, avant ou pendant la formation des hydrates, produire une agitation adaptée à augmenter l'activité dispersante de l'additif.

L'additif pourra comporter des éthanolamides éthoxylés ou non éthoxylés d'acides carboxyliques substitués ou non substitués.

L'additif pourra comporter des esters de polyols et de préférence des esters de polyols comportant au moins trois groupements hydroxy.

L'additif pourra comporter deux composés amphiphiles, le premier de ces composés étant anionique, tel que le dioctylsulfosuccinate de sodium, le deuxième de ces composés étant un composé non-ionique, tel que le monolaurate de sorbitan.

## Revendications

1. Procédé de transport dans une conduite d'un fluide comportant du gaz et de l'eau, et étant dans des conditions où au moins un hydrate se forme, les hydrates étant formés à partir dudit gaz et de ladite eau, caractérisé en ce qu'on injecte dans ledit fluide, avant ou pendant la formation de ou des hydrates, un additif comprenant au moins un composé tensio-actif adapté à réduire la tendance à l'agglomération des hydrates de manière à obtenir un ou des hydrates sous forme dispersée, et en ce qu'on transporte ledit fluide contenant ce ou ces hydrates sous forme dispersée.

2. Procédé selon la revendication 1, dans lequel on utilise une pompe placée sur ladite conduite, caractérisé en ce qu'on injecte l'additif en amont de ladite pompe.

3. Procédé selon l'une des revendications 1 et 2, dans lequel ledit additif a une certaine activité dispersante, caractérisé en ce que sensiblement au moment où les conditions sont réunies pour former un ou des hydrates dans ledit fluide, on produit une agitation adaptée à augmenter l'activité dudit additif.

4. Procédé selon l'une des revendications 1 à 3, caractérisé en ce que ledit fluide comporte des hydrocarbures, tels du pétrole, du gaz naturel ou du gaz de pétrole.

5. Procédé selon l'une des revendications 1 à 4, caractérisé en ce que ledit additif comporte au moins un composé tensio-actif non-ionique.

6. Procédé selon l'une des revendications 1 à 5, caractérisé en ce que ledit additif comprend au moins un composé tensio-actif anionique.

7. Procédé selon l'une des revendications 1 à 6, caractérisé en ce que ledit additif est un composé tensio-actif cationique.

8. Procédé selon l'une des revendication 5 à 7, caractérisé en ce que ledit additif comporte au moins un composé choisi parmi les éthanolamides éthoxylés ou non éthoxylés d'acides carboxyliques substitués ou non substitués et les esters de polyols et d'acides carboxyliques subtitués ou non subtitués.

9. Procédé selon la revendication 8, caractérisé en ce que dans lesdits esters de polyols, le polyol renferme au moins trois groupements hydroxy.

10. Procédé selon l'une des revendications 6 à 9, caractérisé en ce que ledit composé tensio-actif anionique est un sulfonate ou un composé sulfosuccinique.

11. Procédé selon l'une des revendications 1 à 10, caractérisé en ce que ledit additif comprend au moins deux composés tensio-actifs, le premier étant un tensio-actif non-ionique, le second étant un tensio-actif anionique.

12. Procédé selon l'une des revendications 1 à 11, caractérisé en ce que ledit additif est injecté dans ledit fluide en une proportion inférieur à 1 % en poids par rapport à l'eau.

## Claims

1. A process for transporting in a duct a fluid comprising gas and water, and being under conditions where at least one hydrate is formed, the hydrates being formed from said gas and said water, characterized in that, before or during the formation of the hydrate or hydrates, an additive comprising a surfactant adapted to reduce the tendency to agglomeration of the hydrate so as to obtain one or more hydrates in dispersed form, is injected into said fluid, and said fluid containing said hydrate or hydrates in dispersed form is transported.

2. A process according to claim 1, in which a pump is used placed in said duct, characterized in that said additive is injected upstream of said pump.

3. A process according to one of claims 1 and 2, in which said additive has a certain dispersant activity, characterized in that an agitation is provided for increasing the activity of said additive, substantially at the moment when the conditions are present for the formation of one or more hydrates in said fluid.

4. A process according to one of claims 1 to 3, characterized in that said fluid comprises hydrocarbons, such as oil, natural gas or petroleum gas.

5. A process according to one of claims 1 to 4, characterized in that said additive comprises at least one non-ionic tensio-active compound.

6. A process according to one of claims 1 to 5, characterized in that said additive comprises at least one anionic tensio-active compound.

7. A process according to one of claims 1 to 6, characterized in that said additive is a cationic tensio-active compound.

8. A process according to one of claims 5 to 7, characterized in that said additive comprises at least one compound selected from the ethoxylated or non-ethoxylated ethanolamides of substituted or unsubstituted carboxylic acids, and the esters of polyols and substituted or unsubstituted carboxylic acids.

9. A process according to claim 8, characterized in that in said esters of polyols, the polyol comprises at least three hydroxy groups.

10. A process according to one of claims 6 to 9, characterized in that said anionic tensio-active compound is a sulfonate or a sulfosuccinic compound.

11. A process according to one of claims 1 to 10, characterized in that said additive comprises at least two tensio-active compounds, the first one being a non-ionic tensio-active compound, the second one being an anionic tensio-active compound.

12. A process according to one of claims 1 to 11, characterized in that said additive is injected into said fluid in a proportion lower that 1% by weight with respect to water.

## Patentansprüche

1. Ein Verfahren zum Transport einer Gas und Wasser enthaltenden Flüssigkeit durch ein Rohr unter Bedingungen, unter denen wenigstens ein Hydrat gebildet wird, wobei die Hydrate aus dem Gas und dem Wasser gebildet werden, dadurch gekennzeichnet, daß vor oder während der Bildung des Hydrats oder der Hydrate ein Additiv in diese Flüssigkeit eingespritzt wird, das ein zur Verringerung der Agglomerationneigung des Hydrats geeignetes Tensid enthält, um ein oder mehrere Hydrate in dispergierter Form zu erhalten, und diese besagte(s) Hydrat oder Hydrate in dispergierter Form enthaltende Flüssigkeit transportiert wird.

2. Ein Verfahren nach Anspruch 1, in dem eine in dem Rohr angeordnete Pumpe verwendet wird, dadurch gekennzeichnet, daß das Additiv stromaufwärts dieser Pumpe eingespritzt wird.

3. Ein Verfahren nach einem der Ansprüche 1 und 2, worin dieses Additiv eine gewisse dispergierende Wirkung aufweist, dadurch gekennzeichnet, daß eine Bewegung zur Erhöhung der Aktivität dieser Additive, im wesentlichen in dem Moment, in dem die Bedingungen zur Bildung eines oder mehrerer Hydrate in dieser Flüssigkeit vorliegen, bereitgestellt wird.

4. Ein Verfahren nach einem der Ansprüche 1 bis 3, dadurch gekennzeichnet, daß diese Flüssigkeit Kohlenwasserstoffe wie Öl, natürliches Gas oder Erdgas enthält.

5. Ein Verfahren nach einem der Ansprüche 1 bis 4, dadurch gekennzeichnet, daß das Additiv wenigstens eine nichtionische oberflächenaktive Verbindung enthält.

6. Ein Verfahren nach einem der Ansprüche 1 bis 5, dadurch gekennzeichnet, daß das Additiv wenigstens eine anionische oberflächenaktive Verbindung enthält.

7. Ein Verfahren nach einem der Ansprüche 1 bis 6, dadurch gekennzeichnet, daß das Additiv eine kationische oberflächenaktive Verbindung ist.

8. Ein Verfahren nach einem der Ansprüche 5 bis 7, dadurch gekennzeichnet, daß das Additiv wenigstens eine Verbindung, ausgewählt aus den ethoxylierten oder nichtethoxylierten Ethanolamiden von substituierten oder nicht substituierten Carbonsäuren und den Estern von Polyolen und substituierten oder nicht substituierten Carbonsäuren enthält.

9. Ein Verfahren nach Anspruch 8, dadurch gekennzeichnet, daß in diesen Esterpolyolen das Polyol wenigstens drei Hydroxylgruppen enthält.

10. Ein Verfahren nach einem der Ansprüche 6 bis 9, dadurch gekennzeichnet, daß die anionische oberflächenaktive Verbindung ein Sulfonat oder eine Bernsteinsäuresulfon-Verbindung ist.

11. Ein Verfahren nach einem der Ansprüche 1 bis 10, dadurch gekennzeichnet, daß das Additiv wenigstens zwei oberflächenaktive Verbindungen enthält, wobei die erste eine nichtionische oberflächenaktive Verbindung und die zweite eine anionische oberflächenaktive Verbindung ist.

12. Ein Verfahren nach einem der Ansprüche 1 bis 11, dadurch gekennzeichnet, daß das Additiv in die Flüssigkeit in einem Verhältnis von weniger als ein Gewichtsprozent, bezogen auf das Wasser, eingespritzt wird.
